# EUROPEAN PATENT SPECIFICATION

(11) **EP 3 123 986 B1**
(45) Date of publication and mention of the grant of the patent: **14.03.2018**
(21) Application number: 16181861.2
(22) Date of filing: 29.07.2016
(51) Int. Cl.: A61F 5/01

(54) **KNEE BRACE FOR THE TREATMENT OF OSTEOARTHRITIS**
KNIESTÜTZE ZUR BEHANDLUNG VON OSTEOARTHRITIS
ORTHÈSE DE GENOU POUR LE TRAITEMENT DE L'ARTHROSE

(30) Priority: 31.07.2015 IT UB20152714
(43) Date of publication of application: 01.02.2017
(73) Proprietor: Orthoservice AG, 6830 Chiasso (CH)
(72) Inventor: BERNAREGGI, Aldo, 20842 BESANA IN BRIANZA (MB) (IT); TURCONI, Francesco, 20900 MONZA (MB) (IT); ROSSI, Paolo, 6834 MORBIO INFERIORE (CH)
(74) Representative: Branca, Emanuela

(56) References cited:
- WO-A2-2007/041303
- DE-C- 846 895
- DE-U1- 8 517 061
- US-A- 5 277 698
- US-A- 6 110 138

## Description

The present invention relates to a knee brace for the treatment of osteoarthritis, also called gonarthrosis for the specific case of the knee.

Many different types of knee braces for the treatment of gonarthrosis are known, capable of transmitting specific forces to the knee.

In the case of unicompartmental gonarthrosis, a thinning of the cartilage of the knee occurs, in particular on one side of the same, typically the inner side, which causes pain as a result of the direct contact between the bone condyles of femur and tibia, especially when the knee is under load. Hereinafter, "affected side" will be referred to such a side of the joint and "healthy side" to the opposite side.

Knee braces are known that act on three thrust points, placed on opposite sides of the knee, so as to apply a load to the knee adapted to laterally "open" the affected side of the joint subject to deterioration of the cartilage. The distribution of the thrust points on the knee provides that on the healthy side of the joint there is a single thrust point substantially directed towards the joint, whereas two thrust points are arranged on the side of the knee where osteoarthritis occurs, placed above and below the joint, respectively, substantially at the same distance from the joint.

A knee brace with three thrust points is for example described in US 5.277.698 that describes a knee brace provided with an upper half-shell adapted to be constrained to the thigh and a lower half-shell adapted to be constrained to the calf, joined by an articulated rod, adapted to be placed on the affected side of the knee. A strap arranged spiral-wound around the knee with both ends applied to the side of the knee brace bearing the rod, corresponding to the affected side of the knee, to the upper half-shell and to the lower half-shell, respectively, causes the application of the three thrust points, when tightened. In fact, the single thrust point on the healthy side of the knee is determined by the contact point of the strap around the knee whereas the two opposite thrust points on the affected side of the knee are generated by the opposite ends of the articulated rod.

The drawbacks of such a knee brace mainly consist of the wearability of the same and particularly of the difficulty of exercising a suitable tightening force through the strap without causing an undesired rotation of the knee brace.

A solution to this technical problem is suggested by EP 1830756 B1 patent, where the single strap spiral-wound around the knee is doubled, that is to say, two opposing belts spiral-wound around the knee are provided both with their opposite ends applied on the side of the knee brace bearing the rod, corresponding to the affected side of the knee, to the upper half-shell and to the lower half-shell, respectively. The two straps that cross on the healthy side of the knee cause the application of the three thrust points when both tightened. In fact, the single thrust point on the healthy side of the knee is substantially determined by the crossing point of the two straps around the knee whereas the two opposite thrust points on the affected side of the knee are generated by the opposite ends of the articulated rod.

A further knee brace for the treatment of osteoarthritis is disclosed in DE 846895 C1, which is also considered to represent the closest prior art. The presence of two tensioning straps, opposite each other, has the effect of compensating for the rotation of the knee brace when subject to tensioning. However, still remain problems of wearability of the knee brace related to the need of tightening the two straps with a suitable force. Moreover, the more complicated configuration of the straps makes it more difficult to wear the knee brace, in addition to requiring a new adjustment of the same each time.

Moreover, the solutions described above have as a further drawback that the single contact point on the healthy side of the knee is located in a fixed and not modifiable position, given by the constraint point of the straps on the upper and lower half-shells and by the specific anatomy of the patient.

The possibility of adjusting the position of the thrust point both vertically and especially along the circumference of the knee is instead desirable, as this would allow adapting the knee brace as if it were a custom-made brace. In fact, the lateral support on the condyle-tibia must be calibrated for example according to the patient's anatomy, to the diffusion of osteoarthritis, etc., so as to obtain the best possible "opening" of the affected side and to find, also in the test phase upon the first application, the most comfortable configuration for the patient and that better soothes the pain.

The object of the present invention is to make a knee brace for the treatment of osteoarthritis that overcomes the drawbacks mentioned above for the known knee braces.

Another object of the present invention is to make a knee brace for the treatment of osteoarthritis that is easy to wear and to be tensioned.

A further object of the present invention is to make a knee brace for the treatment of osteoarthritis that allows a customized adjustment based on the patient's anatomophysiology.

Another object of the present invention is to make a knee brace for the treatment of osteoarthritis that is particularly simple, functional and cost-effective.

These objects according to the present invention are achieved by making a knee brace for the treatment of osteoarthritis as outlined in claim 1.

Further features are provided in the dependent claims.

The features and advantages of a knee brace for the treatment of osteoarthritis according to the present invention will become more apparent from the following exemplary and non-limiting description, referred to the accompanying schematic drawings, in which:

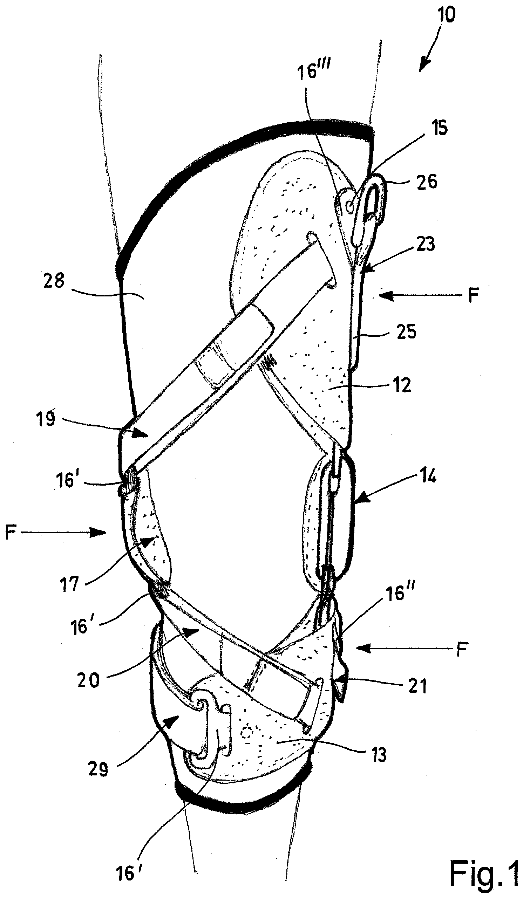

figure 1 is a front elevation view of the knee brace for the treatment of osteoarthritis according to the present invention;

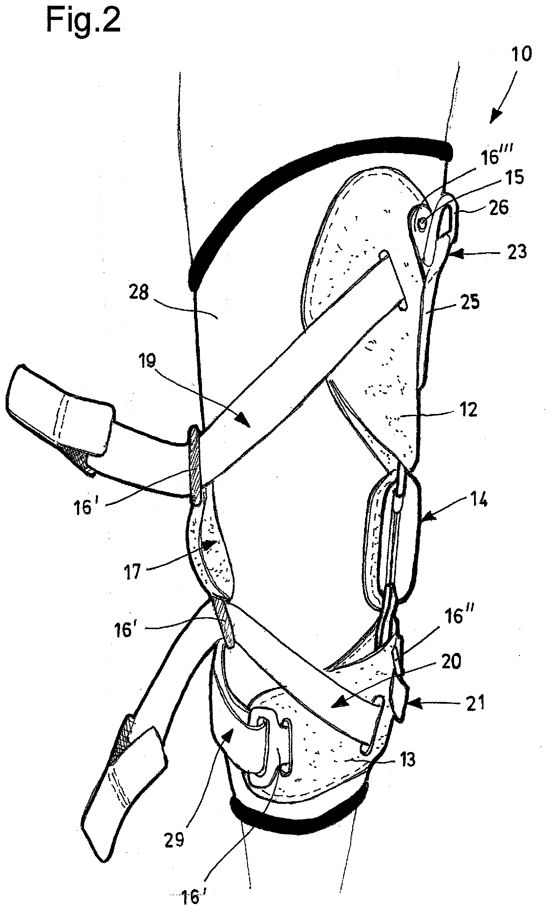

figure 2 shows the knee brace of figure 1 in which the upper and lower transverse front adjustment straps are in the open position;
figure 3 is a lateral view of the knee brace of figure 1 showing the pressure plate placed on the healthy side of the knee;

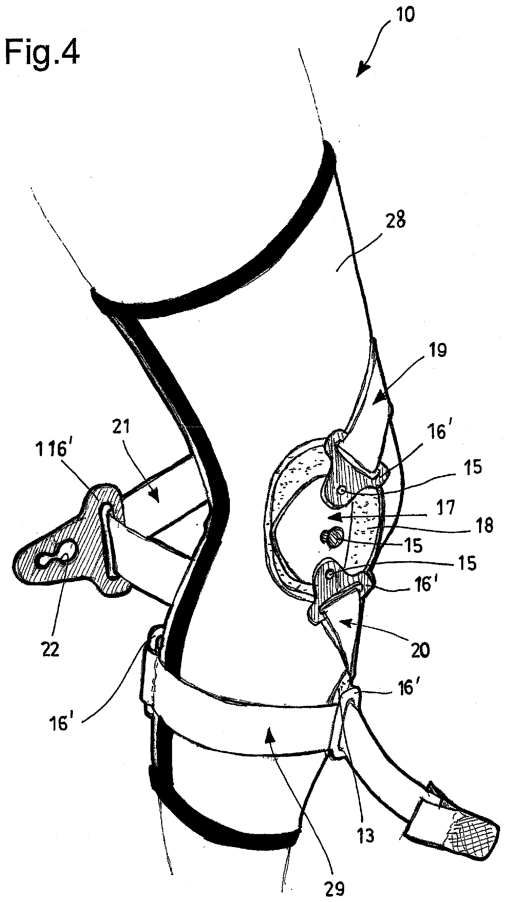

figure 4 shows the knee brace of figure 3 in which the rear tightening strap and the lower strap are in the open position;

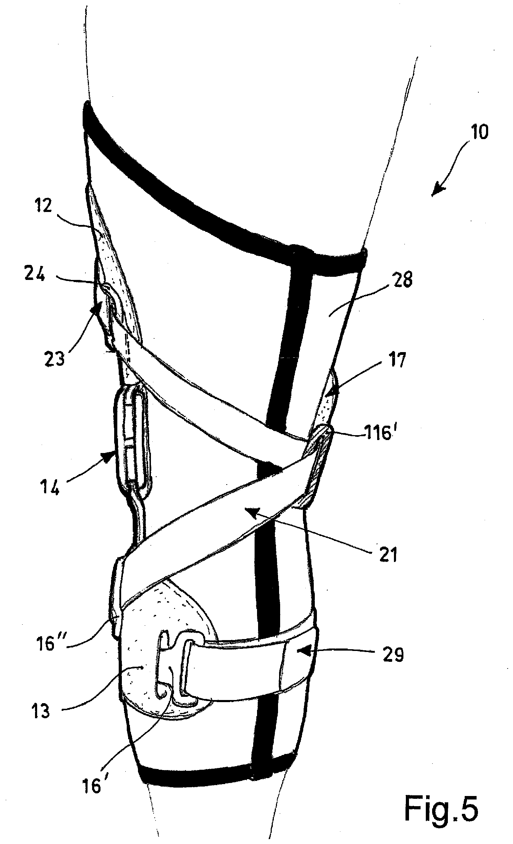

figure 5 is a front rear elevation view of the knee brace for the treatment of osteoarthritis according to the present invention;

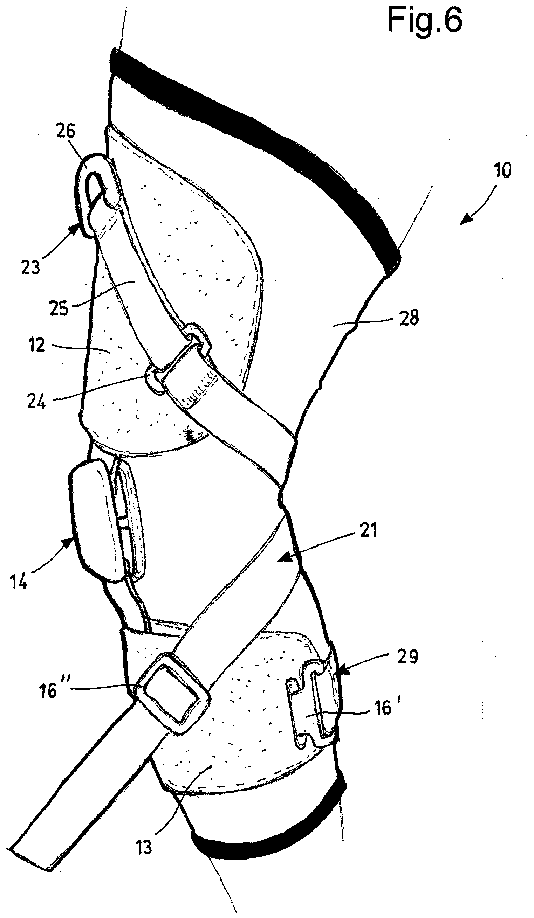

figure 6 is a lateral/rear view of the knee brace object of the invention showing the pulley device and the adjustment loop of the rear tightening strap;

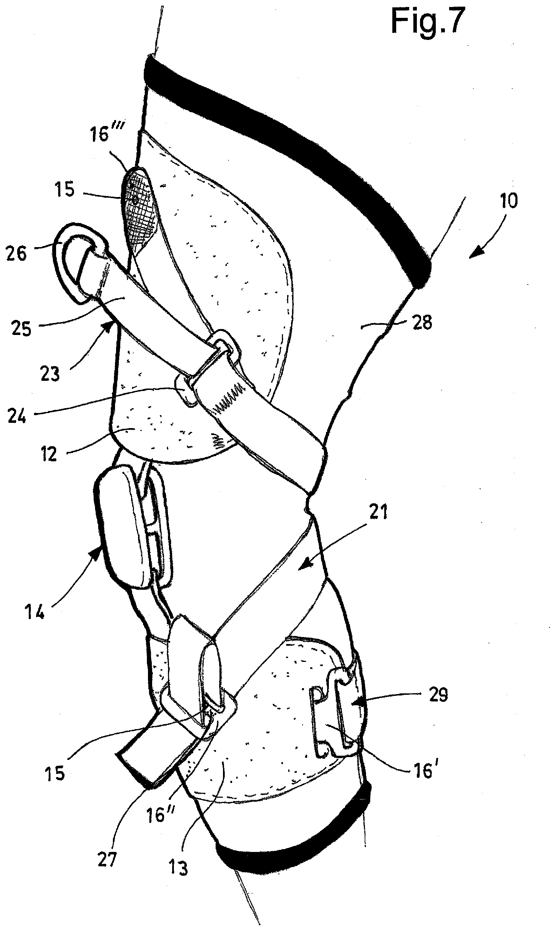

figure 7 shows the ends of the rear tightening strap of the knee brace of figure 6;
figure 8 is a lateral view of a knee brace according to the invention provided with a pressure plate according to an alternative embodiment;
figure 9 is a lateral view of a knee brace according to the invention provided with a pressure plate according to a further embodiment.

With reference to the figures, a knee brace for the treatment of osteoarthritis is shown, globally indicated with reference number 10, comprising an upper half-shell 12, adapted to be constrained laterally to the thigh, and a lower half-shell 13, adapted to be constrained laterally to the calf, joined by an articulated rod 14, adapted to be placed laterally on the affected side of the knee that must be lightened by the load.

The upper 12 and lower 13 half-shells comprise a structural core of plastic material and an outer coating of fabric.

The half-shells 12 and 13 each have a constraining element, not shown, for opposite ends of the articulated rod, preferably adapted to make a removable constraint with the articulated rod 14.

The upper 12 and lower 13 half-shells are further provided with fastening pins 15 for constraining the straps in an articulated manner, for example consisting of pins inserted in the structural core, to which constraining elements 16 for the straps, such as articulated slots 16' and 116', articulated loops or buckles 16" or articulated end plates 16''' are connected in an articulated manner, i.e. allowing a rotation about the axis defined by the pin.

According to an alternative embodiment, the straps may be connected to the half-shells 12 and 13 through the articulated rod 14, being all or in part directly constrained, always in an articulated manner, directly on the ends of the articulated rod 14.

The knee brace 10 further comprises a pressure plate 17 for the lateral support against the knee on the opposite side with respect to the articulated rod 14, which distributes the load on a surface having a plane extension and possibly a surface convexity modifiable as desired according to the patient's needs and anatomy.

The pressure plate 17 comprises a structural portion, for example a structural core of plastic material, possibly provided with an outer fabric coating. On the side facing the knee, the pressure plate is further provided with a padded cushion 18 for improving comfort.

The pressure plate 17 is provided with three fastening pins 15 for constraining the straps in an articulated manner, preferably arranged at the vertices of a triangle for a better distribution of the loads. The fastening pins 15 are connected in an articulated manner to three slots 16' or 116' for the straps, to which a rotation about the axis defined by the pin is allowed for the adaptation of the arrangement of the straps.

The knee brace 10 comprises a strap system mainly consisting of an upper transverse front adjustment strap 19 adjustable in length, connected at opposite ends to the upper half-shell 12 and to the pressure plate 17, respectively, and a lower transverse front adjustment strap 20 adjustable in length, connected at opposite ends to the lower half-shell 13 and to the pressure plate 17, respectively.

Each of the transverse front adjustment straps 19 and 20 comprises a first end fixed to an articulated end plate, not shown, and an opposite end returned in an articulated slot 16' so as to be folded on itself by means of a Velcro element for adjusting the desired length. According to the embodiment shown, the transverse front adjustment straps 19 and 20 are constrained via an articulated end plate to the half-shells 12 and 13 and have the end adjustable in length returned into the articulated slot 16' irremovably connected to the pressure plate 17.

The knee brace 10 further comprises a single rear tightening strap 21, arranged on the rear side of the knee brace 10 according to two diagonals that, starting from the upper 12 and lower 13 half-shells, respectively, converge into the articulated slot 116', removably connectable to the pressure plate 17, for example through a quick release system 22. The articulated slot 116' provided with a quick release system 22 for the rear tightening strap 21 is positioned centrally with respect to the articulated slots 16' for the transverse front adjustment straps 19 and 20, irremovably connected to the pressure plate 17.

A first end of the rear tightening strap 21 is connected to the upper half-shell 12 by means of a pulley tensioning device 23 for tensioning the knee brace 10 capable of demultiplying the forces that must be applied by the user, the tensioning to be achieved being equal. The pulley tensioning device 23 comprises a free slot 24, i.e. not directly constrained to the half-shells 12 and 13 or to the articulated rod 14, to which the end of the rear tightening strap 21 is fixed and in which a tensioning strap 25 is returned. The tensioning strap 25 is provided with an end constrained through an articulated end plate 16''' to the upper half-shell 12 and an opposite end provided with a gripping means 26, such as a ring, for exerting the traction. The tensioning strap 25 is foldable on itself by means of Velcro for a continuous adjustment of the length of the pulley tensioning device 23 and thus of the traction.

An opposite end of the rear tightening strap 21 is instead connected to the lower half-shell 13 through an articulated loop or buckle 16" provided with anti-slip means 27 for the stable adjustment of the length of the rear tightening strap 21. The anti-slip means 27 may consist for example of portions of the loop coated with Velcro or anyway provided with a high friction surface and/or having a particular angle.

At the back, the rear tightening strap 21 describes an upper diagonal between the fastening pin 15 on the upper half-shell 12 in the proximity of the articulated rod 14 and the return slot 116' of the pressure plate 17 and a lower diagonal between the return slot 116' of the pressure plate 17 and the fastening pin 15 on the lower half-shell 13 in the proximity of the articulated rod 14.

Thanks to this arrangement of the rear tightening strap 21, upon the application of the tensioning force, no twist is imparted to the knee brace since the load is distributed in a specular manner on the rear side and on the front side of the knee brace in a corresponding manner between the pressure plate 17 and the two diagonal branches of the rear tightening strap 21, respectively, as well as the two transverse front adjustment straps 19 and 20. The result is an action of substantially sole bending in the frontal plane that tends to laterally "open" the knee in order to relieve the side subject to gonarthrosis.

The position of the pressure plate 17 on the patient's knee, which is a freely floating element joined to the knee brace 10 only via straps 19, 20 and 21, is only determined by the adjustment of the length of the straps related thereto, and therefore is freely adjustable upon fitting the knee brace according to the specific medical needs and to the anatomy of the patient. In particular, the adjustment of the position of the pressure plate can take place both in height and moving forward or backward with respect to the joint.

Generally, the adjustment of the position of the pressure plate 17 is carried out so as to place the single thrust point at the pressure plate 17 about symmetrically in "vertical" with respect to the two opposite thrust points at the ends of the articulated rod 14, as shown with arrows F in the figures. Of greater importance is instead the adjustment along the circumference of the knee that allows adapting the knee brace as if it were a custom-made brace. In fact, the lateral support on the condyle-tibia must be calibrated for example according to the patient's anatomy, to the diffusion of osteoarthritis, etc., so as to obtain the best possible "opening" of the affected side and to find, also in the test phase upon the first application, the most comfortable configuration for the patient and that better soothes the pain.

According to a preferred embodiment, the knee brace 10 also includes a fabric sleeve 28, placed on the side of the knee brace facing the patient's leg, which protects the leg from the friction with the parts of the knee brace 10, in particular during the tightening operation. The fabric sleeve 28, preferably elastic, can be made as a closed tubular sock or possibly also open longitudinally. Moreover, the fabric sleeve can be provided or not with a patellar hole.

The knee brace, according to the preferred embodiment shown by way of example, comprises a lower strap 29, adjustable in length, connected in an articulated manner through slots 16' between two opposite points of the lower shell 12 for the circumferential clamping of the calf.

Optionally, the knee brace according to the invention may not have such a lower strap or it may also comprise an upper strap, adjustable in length, connected between two opposite points of the upper shell for the circumferential clamping of the thigh, not shown.

The knee brace 10 according to the invention, upon the first application on a patient, provides for the adjustment of the upper 19 and lower 20 transverse front adjustment straps, as well as of the maximum length of the rear tightening strap 21 for defining the desired position of the pressure plate 17.

After the first application of the knee brace 10, and for all subsequent applications, the knee brace 10 is tensioned by means of the pulley tensioning device 23 of the rear tightening strap 21. This operation must be carried out with the knee flexed, when the rear portion of the knee brace is at the minimum length. In this way, the knee brace 10 exerts an even greater force when extending the knee, since the rear portion of the knee brace tends to "stretch". In fact, it is important that the knee brace exerts more strength with the knee extended and thus when standing, because the pain given by gonarthrosis occurs when standing and the articular surfaces are under the body weight's pressure. Instead, when sitting, the knee is relieved and therefore less force is required because it is not necessary to "distance" the joint surfaces.

With the knee brace 10 worn, the force is exerted by the pressure plate 17, that is opposite the articulated rod 14, and by the two points at the ends of the articulated rod 14, so as to decrease the contact force between the femoral condyle and the lateral-presser portion of tibia, that is to say, to try to laterally "open" the knee joint as indicated by arrows F.

The quick release system 22 of the articulated slot 116' removably connected to the pressure plate 17, in which the rear tightening strap 21 is returned, allows opening and wearing the knee brace without changing the adjustments of the lengths of the straps.

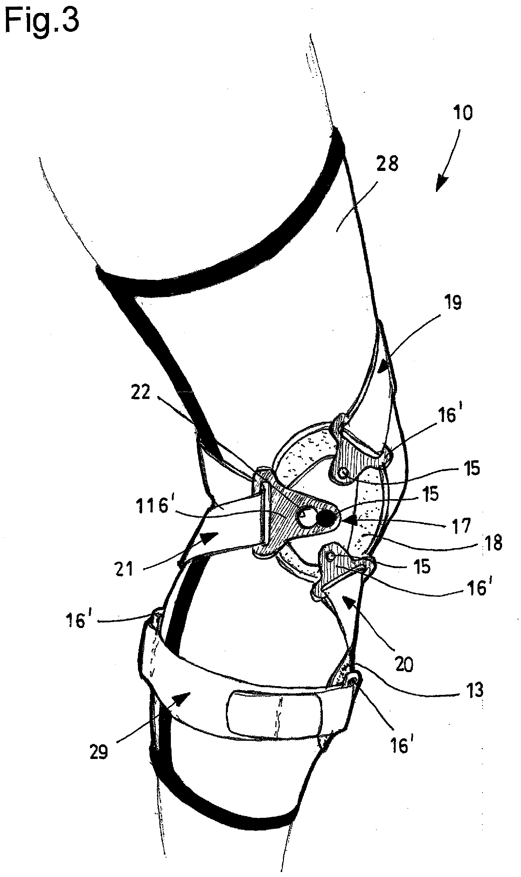

According to a first embodiment of the knee brace 10, for example shown in figure 3, the pressure plate 17 is only constrained to the three fastening pins 15 and, for a proper application of the corrective forces on the knee, through the correct adjustment of the transverse front adjustment straps 19 and 20 and of the rear tightening strap 21, it is positioned on the patient so as to laterally act on the knee at each application of the knee brace 10.

In order to facilitate the positioning of the pressure plate 17 laterally on the knee, the pressure plate 17, according to alternative embodiments, can be stably constrained to the fabric sleeve 28 in a lateral position, so as to be in the correct positioning when the knee brace is worn, and guide the adjustment of the straps 19, 20, 21.

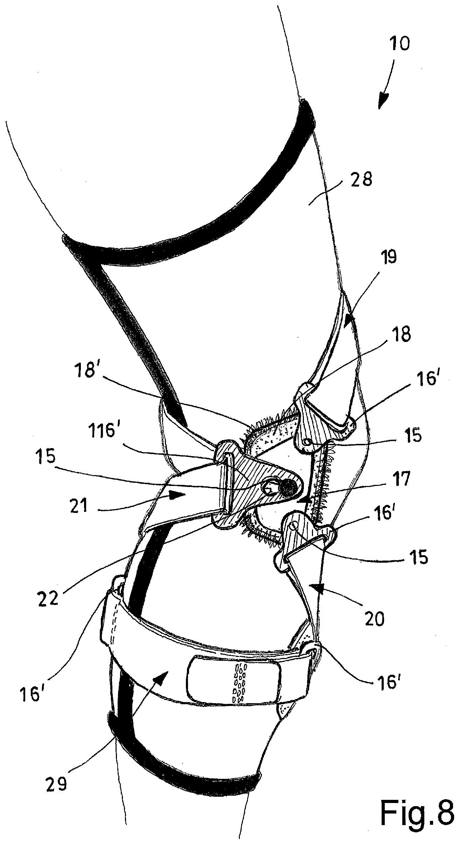

According to an embodiment shown in figure 8, this constraint can be made through a sewing 18' between the sleeve 28 and for example the padded cushion 18, in turn integrally joined to the pressure plate 17.

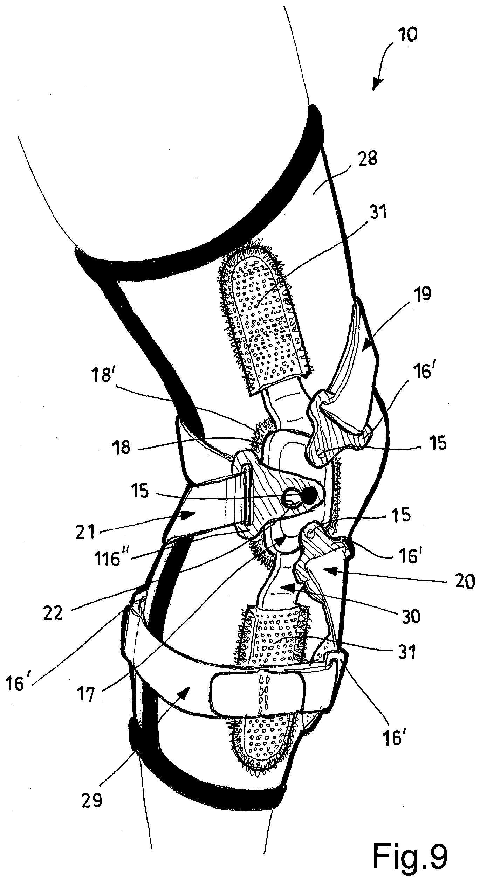

According to a further embodiment, the constraint between the pressure plate 17 and the sleeve 28 is obtained by making the pressure plate 17 integral to a second articulated rod 30, arranged on the opposite side of the knee brace 10 with respect to articulated rod 14. The pressure plate 17 is made integral to the second articulated rod 30, for instance by making the pressure plate 17 coincide with the articulation portion of the second articulated rod 30, to which the fastening pins 15 are for example directly connected, as shown in figure 9. The second articulated rod 30 is in turn stably constrained to the sleeve 28, for example through the provision of pockets 31 sewed on the sleeve 28 itself for the insertion of the opposite ends of the second articulated rod 30. In this embodiment the padded cushion 18 can be directly applied to the sleeve 28 through a sewing 18' and can be itself constrained to the second articulated rod 30, for instance through the provision of constraining means such as Velcro®, not shown, in order to contribute to the positioning of the second articulated rod 30 with respect to the sleeve 28.

The knee brace for the treatment of osteoarthritis subject-matter of the present invention has the advantage of allowing the positioning of the pressure plate in height and along the circumference of the knee to adjust the pressure point as desired.

Moreover, the knee brace of the invention advantageously provides for carrying out the adjustments of the lengths of the straps just during the first application. Moreover, it advantageously comprises a quick release system of the pressure plate to open the knee brace in order to easily wear it.

The knee brace of the invention is advantageously tightened by means of a pulley device that demultiplies the force that must be applied, the tensioning of the knee brace being the same.

A further advantage consists in that the tightening operation can be carried out with the knee flexed when the rear portion of the knee brace is at the minimum length and in that upon the knee extension, the knee brace will exert an even greater force.

Several changes and variations may be made to the knee brace for the treatment of osteoarthritis thus conceived, all falling within the invention; moreover, all details can be replaced with technically equivalent elements. In the practice, the materials used as well as the sizes, can be whatever, according to the technical requirements.

## Claims

1. Knee brace for the treatment of osteoarthritis comprising an upper half-shell (12) and a lower half-shell (13), joined by an articulated rod (14), as well as a pressure plate (17) for the lateral support against the knee on the opposite side with respect to said articulated rod (14), said knee brace comprises a strap system comprising an upper transverse front adjustment strap (19), adjustable in length, connected at opposite ends to said upper half-shell (12) and to said pressure plate (17), respectively, and a lower transverse front adjustment strap (20), adjustable in length, connected at opposite ends to said lower half-shell (13) and to said pressure plate (17), respectively, as well as a sole rear tightening strap (21), adjustable in length and provided with a pulley tensioning device (23), arranged on the rear side of the knee brace (10) along two diagonals that starting from said upper (12) and said lower (13) half-shells, respectively, converge to an articulated slot (116') of said pressure plate (17), wherein said straps are movably or unmovably connected to said half-shells (12, 13) and to said pressure plate (17) exclusively in an articulated manner through constraining elements (16), such as articulated slots (16', 116') and/or articulated loops or buckles (16") and/or articulated end plates (16'''), adapted to allow the rotation of the straps.

2. Knee brace according to claim 1, **characterized in that** said upper (12) and said lower (13) half-shells and said pressure plate (17) are provided with fastening pins (15) for constraining in an articulated manner, either movably or unmovably, the hooking means (16) of said straps, said fastening pins (15) being preferably inserted in a structural core.

3. Knee brace according to claim 2, **characterized in that** said pressure plate (17) is provided with three fastening pins (15) arranged at the vertices of a triangle for a better distribution of the loads.

4. Knee brace according to claim 1 or 2, **characterized in that** said transverse front adjustment straps (19 and 20) are constrained at one end to said half-shells (12 and 13) through said articulated end plate (16''') and have the opposite end adjustable in length returned in said articulated slot (16') unmovably connected to said pressure plate (17).

5. Knee brace according to any one of the preceding claims, **characterized in that** said pulley tensioning device (23) comprises a tensioning strap (25) provided with an end constrained by means of an articulated end plate (16''') to said upper half-shell (12) and with an opposite end provided with a gripping means (26), said tensioning strap (25) being returned in a free slot (24) to which one end of the rear tightening strap (21) is fixed, said tensioning strap (25) being foldable on itself by means of Velcro for a continuous adjustment of traction.

6. Knee brace according to any one of the preceding claims, **characterized in that** one end adjustable in length of said rear tightening strap (21) is connected to said lower half-shell (13) through an articulated loop (16"), preferably provided with anti-slip means (27) for the stabilization of the length adjustment of said rear tightening strap (21).

7. Knee brace according to any one of the preceding claims, **characterized in that** said articulated slot (116') for the movable connection of said rear tightening strap (21) to said pressure plate (17) is provided with a quick release system (22).

8. Knee brace according to any one of the preceding claims, **characterized in that** said articulated slot (116') for the movable connection of said rear tightening strap (21) to said pressure plate (17) is positioned centrally with respect to said unmovable articulated slots (16') for said transverse front adjustment straps (19 and 20).

9. Knee brace according to any one of the preceding claims, **characterized in that** said strap system further comprises at least one lower strap (29), adjustable in length, connected in an articulated manner between two opposite points of said lower shell (12) for the circumferential clamping of the calf.

10. Knee brace according to any one of the preceding claims, **characterized in that** it comprises a fabric sleeve (28), arranged on the side of the knee brace (10) facing the patient's leg.

11. Knee brace according to claim 10, **characterized in that** said pressure plate (17) is stably constrained to said fabric sleeve (28).

12. Knee brace according to claim 11, **characterized in that** said pressure plate (17) is stably constrained to said fabric sleeve (28) through a sewing (18').

13. Knee brace according to claim 11, **characterized in that** said pressure plate (17) is integral to a second articulated rod (30), opposite ends of said second articulated rod (30) being stably constrained to said sleeve (28).

## Patentansprüche

1. Knieorthese für die Behandlung von Arthrose, umfassend eine obere Halbschale (12) und eine untere Halbschale (13), die durch eine Gelenkstange (14) verbunden werden, sowie eine Druckplatte (17) für den Seitenhalt gegen das Knie auf der bezüglich der Gelenkstange (14) gegenüberliegenden Seite, wobei die Knieorthese ein Gurtensystem umfasst, das einen in der Länge einstellbaren oberen querlaufenden vorderen Einstellgurt (19), der an gegenüberliegenden Enden jeweils mit der oberen Halbschale (12) und der Druckplatte (17) verbunden wird, und einen in der Länge einstellbaren unteren querlaufenden vorderen Einstellgurt (20) umfasst, der an gegenüberliegenden Enden jeweils mit der unteren Halbschale (13) und der Druckplatte (17) verbunden wird, sowie einem einzigen hinteren Spanngurt (21), der in der Länge einstellbar ist und mit einer Riemenspannvorrichtung (23) bereitgestellt wird, die auf der Rückseite der Knieorthese (10) entlang zweier Diagonalen angeordnet wird, die jeweils beginnend von der oberen (12) und der unteren (13) Halbschale zu einem Gelenkschlitz (116') der Druckplatte (17) zusammenlaufen, wobei die Gurten mit den Halbschalen (12, 13) und der Druckplatte (17) ausschließlich in einer gelenkigen Weise über Begrenzungselemente (16) beweglich oder unbeweglich, wie z.B. Gelenkschlitze (16', 116') und/oder Gelenkschlaufen oder Schnallen (16") und/oder Gelenkendplatten (16''') verbunden werden, die angepasst werden, um die Drehung der Gurten zu ermöglichen.

2. Knieorthese nach Anspruch 1, **dadurch gekennzeichnet, dass** die obere (12) und die untere (13) Halbschale und die Druckplatte (17) mit entweder beweglichen oder unbeweglichen Befestigungsstiften (15) zum Begrenzen der Befestigungsmittel (16) der Gurten in einer gelenkigen Weise bereitgestellt werden, wobei die Befestigungsstifte (15) vorzugsweise in einen strukturellen Kern eingefügt werden.

3. Knieorthese nach Anspruch 2, **dadurch gekennzeichnet, dass** die Druckplatte (17) mit drei Befestigungsstiften (15) bereitgestellt wird, die an den Eckpunkten eines Dreiecks für eine bessere Verteilung der Lasten angeordnet werden.

4. Knieorthese nach Anspruch 1 oder 2, **dadurch gekennzeichnet, dass** die querlaufenden vorderen Einstellgurte (19 und 20) an einem Ende zu den Halbschalen (12 und 13) durch die Gelenkendplatte (16''') begrenzt werden und das gegenüberliegende Ende in der Länge einstellbar haben, das in den Gelenkschlitz (16') zurückgeführt wird, der mit der Druckplatte (17) unbeweglich verbunden wird.

5. Knieorthese nach einem der vorhergehenden Ansprüche, **dadurch gekennzeichnet, dass** die Riemenspannvorrichtung (23) einen Spanngurt (25) umfasst, der mit einem mittels einer Gelenkendplatte (16''') begrenzten Ende an der oberen Halbschale (12) bereitgestellt und mit einem gegenüberliegenden Ende mit einem Griffmittel (26) bereitgestellt wird, wobei der Spanngurt (25) in einen freien Schlitz (24) zurückgeführt wird, an den ein Ende des hinteren Spanngurtes (21) befestigt wird, wobei der Spanngurt (25) auf sich selbst mittels Klettverschluss für eine kontinuierliche Einstellung der Zugkraft klappbar ist.

6. Knieorthese nach einem der vorhergehenden Ansprüche, **dadurch gekennzeichnet, dass** ein in der Länge einstellbares Ende des hinteren Spanngurtes (21) mit der unteren Halbschale (13) durch eine Gelenkschlaufe (16") verbunden wird, die vorzugsweise mit Antirutschmitteln (27) für die Stabilisierung der Längeneinstellung des hinteren Spanngurtes (21) bereitgestellt wird.

7. Knieorthese nach einem der vorhergehenden Ansprüche, **dadurch gekennzeichnet, dass** der Gelenkschlitz (116') für die bewegliche Verbindung des hinteren Spanngurtes (21) zu der Druckplatte (17) mit einem Schnellwechselsystem (22) bereitgestellt wird.

8. Knieorthese nach einem der vorhergehenden Ansprüche, **dadurch gekennzeichnet, dass** der Gelenkschlitz (116') für die bewegliche Verbindung des hinteren Spanngurtes (21) zu der Druckplatte (17) bezüglich der unbeweglichen Gelenkschlitze (16') für die querlaufenden vorderen Einstellgurte (19 und 20) zentral positioniert wird.

9. Knieorthese nach einem der vorhergehenden Ansprüche, **dadurch gekennzeichnet, dass** das Gurtensystem des Weiteren mindestens einen in der Länge einstellbaren unteren Gurt (29) umfasst, der in einer gelenkigen Weise zwischen zwei gegenüberliegenden Punkten der unteren Schale (12) für das umlaufende Einspannen der Wade angeschlossen wird.

10. Knieorthese nach einem der vorhergehenden Ansprüche, **dadurch gekennzeichnet, dass** sie eine Gewebehülse (28) umfasst, die auf der Seite der Knieorthese (10) angeordnet wird, die dem Bein des Patienten gegenübersteht.

11. Knieorthese nach Anspruch 10, **dadurch gekennzeichnet, dass** die Druckplatte (17) an der Gewebehülse (28) stabil begrenzt wird.

12. Knieorthese nach Anspruch 11, **dadurch gekennzeichnet, dass** die Druckplatte (17) an der Gewebehülse (28) durch ein Nähen (18') stabil begrenzt wird.

13. Knieorthese nach Anspruch 11, **dadurch gekennzeichnet, dass** die Druckplatte (17) mit einer zweiten Gelenkstange (30) einstückig ist, wobei gegenüberliegende Enden der zweiten Gelenkstange (30) an der Hülse (28) stabil begrenzt werden.

## Revendications

1. Orthèse de genou pour le traitement de l'arthrose comprenant une demi-coque supérieure (12) et une demi-coque inférieure (13), réunies par une tige articulée (14), ainsi qu'une plaque de pression (17) pour le support latéral contre le genou du côté opposé par rapport à ladite tige articulée (14), ladite orthèse de genou comprend un système de sangle comprenant une sangle de réglage avant transversale supérieure (19), réglable en longueur, reliée aux extrémités opposées à ladite demi-coque supérieure (12) et à ladite plaque de pression (17), respectivement, et une sangle de réglage avant transversale inférieure (20), réglable en longueur, reliée aux extrémités opposées à ladite demi-coque inférieure (13) et à ladite plaque de pression (17), respectivement, ainsi qu'une seule sangle de serrage arrière (21), réglable en longueur et pourvue d'un dispositif de tension à poulie (23), agencée sur le côté arrière de l'orthèse de genou (10) le long de deux diagonales qui partant de ladite demi-coque supérieure (12) et de ladite demi-coque inférieure (13), respectivement, convergent vers une fente articulée (116') de ladite plaque de pression (17), dans laquelle lesdites sangles sont reliées de manière mobile ou non auxdites demi-coques (12, 13) et à ladite plaque de pression (17) exclusivement de manière articulée à travers des éléments de contrainte (16), tels que des fentes articulées (16', 116') et/ou des boucles articulées (16") et/ou des plaques d'extrémité articulées (16'''), adaptées pour permettre la rotation des sangles.

2. Orthèse de genou selon la revendication 1, **caractérisée en ce que** lesdites demi-coques supérieure (12) et inférieure (13) et ladite plaque de pression (17) sont munies de goupilles de fixation (15) pour contraindre de manière articulée, mobile ou non, les moyens d'accrochage (16) des dites sangles, lesdites goupilles de fixation (15) étant de préférence insérées dans un noyau structurel.

3. Orthèse de genou selon la revendication 2, **caractérisée en ce que** ladite plaque de pression (17) est pourvue de trois goupilles de fixation (15) disposées aux sommets d'un triangle pour une meilleure répartition des charges.

4. Orthèse de genou selon la revendication 1 ou 2, **caractérisée en ce que** lesdites sangles de réglage avant transversales (19 et 20) sont contraintes à une extrémité auxdites demi-coques (12 et 13) au moyen de ladite plaque d'extrémité articulée (16''') et dont l'extrémité opposée réglable en longueur est retournée dans ladite fente articulée (16') reliée de façon non-mobile à ladite plaque de pression (17).

5. Orthèse de genou selon l'une quelconque des revendications précédentes, **caractérisée en ce que** ledit dispositif de tension à poulie (23) comprend une sangle de tension (25) munie d'une extrémité contrainte au moyen d'une plaque d'extrémité articulée (16''') à ladite demi-coque supérieure (12) et avec une extrémité opposée pourvue d'un moyen de préhension (26), ladite sangle de tension (25) étant retournée dans une fente libre (24) à laquelle une extrémité de la sangle de serrage arrière (21) est fixée, ladite sangle de tension (25) étant repliable sur elle-même au moyen de velcro pour un réglage continu de la traction.

6. Orthèse de genou selon l'une quelconque des revendications précédentes, **caractérisée en ce qu'**une extrémité réglable en longueur de ladite sangle de serrage arrière (21) est reliée à ladite demi-coque inférieure (13) par une boucle articulée (16"), de préférence munie de moyens antidérapants (27) pour la stabilisation du réglage en longueur de ladite sangle de serrage arrière (21).

7. Orthèse de genou selon l'une quelconque des revendications précédentes, **caractérisée en ce que** ladite fente articulée (116') pour la connexion mobile de ladite sangle de serrage arrière (21) à ladite plaque de pression (17) est munie d'un système à largage rapide (22).

8. Orthèse de genou selon l'une quelconque des revendications précédentes, **caractérisée en ce que** ladite fente articulée (116') de connexion mobile de ladite sangle de serrage arrière (21) à ladite plaque de pression (17) est positionnée centralement par rapport auxdites fentes articulées non mobiles (16') pour lesdites sangles de réglage avant transversales (19 et 20).

9. Orthèse de genou selon l'une quelconque des revendications précédentes, **caractérisée en ce que** ledit système de sangle comprend en outre au moins une sangle inférieure (29), réglable en longueur, reliée de manière articulée entre deux points opposés de ladite coque inférieure (12) pour le serrage circonférentiel du mollet.

10. Orthèse de genou selon l'une quelconque des revendications précédentes, **caractérisée en ce qu'**elle comprend un manchon en tissu (28), disposé du côté de l'orthèse de genou (10) en regard de la jambe du patient.

11. Orthèse de genou selon la revendication 10, **caractérisée en ce que** ladite plaque de pression (17) est contrainte de manière stable audit manchon en tissu (28).

12. Orthèse de genou selon la revendication 11, **caractérisée en ce que** ladite plaque de pression (17) est contrainte de manière stable audit manchon en tissu (28) au moyen d'une couture (18').

13. Orthèse de genou selon la revendication 11, **caractérisée en ce que** ladite plaque de pression (17) est solidaire d'une deuxième tige articulée (30), les extrémités opposées de ladite deuxième tige articulée (30) étant contraintes de manière stable audit manchon (28).
